# EUROPÄISCHE PATENTSCHRIFT

(11) **EP 0 368 974 B1**
(45) Veröffentlichungstag und Bekanntmachung des Hinweises auf die Patenterteilung: **03.02.1993**
(21) Anmeldenummer: 89906082.6
(22) Anmeldetag: 24.05.1989
(51) Int. Cl.: B01D 69/10, B01D 67/00, B29C 63/34, B29C 63/20

(54) **VERFAHREN ZUR HERSTELLUNG VON ROHRMEMBRANEN AUS SILIKONKAUTSCHUK MIT EINER MECHANISCHEN WANDVERSTÄRKUNG, AUSBILDUNG VON ROHR MEMBRANEN MIT EINER MECHANISCHEN WANDVERSTÄRKUNG UND ANWENDUNG DIESER ROHRMEMBRAN**
PROCESS FOR PRODUCING SILICON CAOUTCHOUC TUBE MEMBRANES WITH MECHANICAL WALL REINFORCEMENT, DEVELOPMENT OF TUBE MEMBRANES WITH MECHANICAL WALL REINFORCEMENT AND THEIR USE
PROCEDE POUR PRODUIRE DES TUBES A MEMBRANES EN CAOUTCHOUC AU SILICONE AVEC RENFORCEMENT MECANIQUE DES PAROIS, FORMATION DESDITS TUBES A MEMBRANES ET LEUR APPLICATION

(30) Priorität: 24.05.1988 DE 3817560
(43) Veröffentlichungstag der Anmeldung: 23.05.1990
(73) Patentinhaber: BRÄUTIGAM, Hans-Jürgen, D-21077 Hamburg (DE)
(72) Erfinder: BRÄUTIGAM, Hans-Jürgen, D-21077 Hamburg (DE)
(74) Vertreter: Schmidt-Bogatzky, Jürgen, Dr. Ing. Huth, Dietrich & Partner
(86) Internationale Anmeldenummer: EP8900578
(87) Internationale Veröffentlichungsnummer: WO8911318

## Beschreibung

Die Erfindung betrifft eine Rohrmembran aus Silikonkautschuk mit einer mechanischen Wandverstärkung und das Verfahren zu deren Herstellung.

Zur Durchführung von Gasaustauschprozessen wie z. B. Sauerstoffeintrag in Nährmedien bei Fermentationen ist die Verwendung von Membranen aus gasdurchlässigem Silikonkautschuk bekannt. Der Gasaustausch erfolgt blasenfrei als Folge von Lösungs- und Diffusionsprozessen im porenfreien Kunststoffmaterial. Die Austauschgeschwindigkeit ist um so größer, je größer die im Reaktor installierte Membranfläche und die Gaspartialdruckdifferenz zwischen den der Membran anliegenden Phasen sind und um so dünner die Membran ist. Sofern flüssige Phasen beteiligt sind, ist darüberhinaus eine gute Anströmung der Membranoberfläche anzustreben. Wegen der herausragenden Gasdurchlässigkeit ist Silikonkautschuk im Vergleich zu anderen Polymerwerkstoffen besonders gut geeignet. In der DE-C-29 40 446 ist bereits vorgeschlagen worden, zur Sauerstoffversorgung von Kulturen in Fermentern dünnwandige Silikonschläuche zu verwenden. Bei einem Schlauchdurchmesser von nur etwa 5 mm und einer Wandstärke von ca. 0,5 mm sind mit diesen Silikonschläuchen Sauerstoffaustauschraten von etwa 50 mg0₂/h je Meter Schlauch erzielbar, da die Silikonschläuche eine begrenzte mechanische Festigkeit aufweisen und mit Überdrücken bis maximal 1 bar belastbar sind. Wegen der begrenzten mechanischen Festigkeit von Silikonschläuchen ist die Möglichkeit der Druckerhöhung und Durchmesservergröberung begrenzt, so daß mit Silikonschläuchen nur relativ geringe Sauerstoffaustauschraten möglich sind. Um diesen Nachteil zu beseitigen, ist nach der DE-A-31 22 186 bereits vorgeschlagen worden, zur Be- und Entgasung von Flüssigkeiten Silikonmembranen mit Stützgeweben aus Edelstahl herzustellen, bei denen das Stützgewebe elektrostatisch mit einem Polymerisat beschichtet wird. Die Membrandicke liegt hierbei zwischen 1 µ m und 500 µ m. Bei den so hergestellten Silikonmembranen besteht der Nachteil, daß bei der elektrostatischen Beschichtung Fehlstellen auftreten können, die zur Funktionsunfähigkeit der Membranen führen. Nachteilig bei der Herstellung von Silikonmembranen für Gasaustauschprozesse durch elektrostatische Beschichtung von metallischen Stützgeweben, die in Kett- und/oder Schußrichtung abwechselnd einfach oder doppelt gewebt sind, ist die unerläßliche, kostenintensive Vorbereitung der Gewebe, um einen gleichmäßig dünnen und löcherfreien Film zu erzielen. Die mit der Durchführung einer fehlerfreien elektrostatischen Beschichtung verbundenen Schwierigkeiten haben dazu geführt, daß sich derartig hergestellte Silikonmembranen nicht durchsetzen konnten.

Durch die DE-A-35 44 382 ist als Alternative vorgeschlagen worden, Silikonschläuche zu verwenden, die durch eine flexible Gewebeverstärkung druckbeaufschlagbar ausgebildet werden und somit höhere Gasaustauschraten ermöglichen. Bei Sauerstoffwurden mit diesen Schläuchen Eintragsraten von 150 mg0₂/h je Meter Schlauch erzielt. Trotzdem werden bei Prozessen mit hohen Umsatzraten und in Reaktoren mit einem Volumen von mehr als 50 Litern noch große Schlauchmengen benötigt, deren Installation im Reaktor zusätzliche und technisch aufwendige Haltevorrichtungen erfordert. Üblich sind derzeit Schlauchmembranen mit einem Außendurchmesser von etwa 5 mm und einer Wandstärke von 0,5 mm. Die Gewebeverstärkung liegt entweder auf der Schlauchaußenwand oder zwischen zwei miteinander verklebten Silikonschläuchen. Bei der Herstellung derartiger verstärkter Silikonschläuche wird stets von einem unverstärkten ausvulkanisierten Silikonschlauch ausgegangen. Im Falle der außenliegenden Verstärkung wird in einem zweiten Arbeitsschritt die Gewebeummantelung nach bekannten Verfahren aufgebracht. Die Schlauchvariante mit der integrierten Gewebeverstärkung wird erhalten, indem man einen außenummantelten Silikonschlauch in einem weiteren Arbeitsgang durch einen Extruder führt, der einen ca. 0,1 mm bis 0,2 mm dicke Silikonschicht auf den Schlauch bzw. die Gewebeverstärkung aufspritzt. Der aufgespritzte Silikonaußenschlauch wird danach in bekannter Weise vulkanisiert. Derartige verstärkte Silikonschlauchmembransysteme zeichnen sich gegenüber den unverstärkten Membransystemen durch höhere Gasaustauschraten aus. Hierdurch kann für die Durchführung eines vorgegebenen Austauschprozesses die notwendige Membranfläche reduziert werden, was Kosten einspart. Sofern jedoch Gasaustauschprozesse mit hohen Umsatzraten in großen Apparaturen durchgeführt werden müssen, werden große Schlauchmengen benötigt, deren Installation in dem betreffenden Apparat zusätzliche kostensteigernde Haltevorrichtungen erfordert. So müssen beispielsweise in einem Fermenter mit einem Arbeitsvolumen von 1 m³ zur Kultivierung tierischer Zellkulturen mit einem Sauerstoffbedarf von 100 g0₂/h 666 m Silikonschlauch installiert werden. Um die Schlauchlänge und damit den Schlauchbedarf sowie den technischen Aufwand zu verringern, ist es wünschenswert, Schlauchmembranen mit einem gröberen Außendurchmesser und damit einer höheren spezifischen Gaseintragsleistung zu verwenden. Die Herstellung derartiger Silikonschläuche mit einem Auß endurchmesser von z. B. 20 mm und mehr ist technisch jedoch nur möglich, wenn gleichzeitig die Wanddicke des Silikonschlauches, auf dem die Gewebeverstärkung aufgebracht werden muß, auf ca. 2 mm bis 3 mm vergrößert wird. Derartig dickwandige Silikonschläuche ermöglichen jedoch nur geringe Gasaustauschraten und sind daher für eine technische Anwendung uninteressant. Darüber hinaus würden derartige Silikonschläuche wegen der erforderlichen groben Silikonkautschukmengen erhebliche Schlauchkosten verursachen. Wünschenswert ist weiterhin der Verzicht auf Halte- und Stützvorrichtungen. Zum einen können sich hierstörende Ablagerungen bilden , zum anderen erhöht sich die Störanfälligkeit, da bei Druckbeaufschlagung die Schlauchmembran an den Auflagestellen beschädigt werden kann.

Die Aufgabe der Erfindung besteht darin, eine Rohrmembran aus Silikonkautschuk mit einer mechanischen Wandverstärkung und ein Verfahren zu deren Herstellung so zu gestalten, daß Rohrmembranen bei dünnwandiger Ausbildung einen wesentlich gröberen Durchmesser als bekannte Silikonschläuche aufweisen und so formstabil sind, daß sie beim Einsatz auch in groben Fermentern keine Stütz- und Haltevorrichtungen erfordern.

Erfindungsgemäß erfolgt die Lösung der Aufgabe bezüglich der Rohrmembran durch die kennzeichnenden Merkmale des Anspruchs 1 und bezüglich des Verfahrens alternativ durch die kennzeichnenden Merkmale der Ansprüche 11 oder 12 oder 13.

Nach der Erfindung werden formsteife ggf. vorgefertigte Hohlzylinder aus Metall, Metallgewebe, Blech oder Kunststoffen mit Löchern oder anderen Durchbrechungen in der Wand verwendet, auf die die eigentliche dünne und gasdurchlässige Silikonkautschukmembran als Folie aufgebracht wird. Die Folie kann durch herkömmliche Tauch- oder Extrusionsverfahren mit anschließender Vulkanisation direkt auf dem Hohlzylinder hergestellt werden. Es ist auch die Verwendung handelsüblicher verstärkter oder unverstärkter Flachmembranen aus Silikonkautschuk möglich, die um den Hohlzylinder gelegt und an der Längsnaht miteinander verklebt werden. Vorteilhaft kann auch die Verwendung vorgefertigter dünnwandiger Silikonschläuche sein, die über den Hohlzylinder gezogen werden. Als formsteife Hohlzylinder eignen sich auch glatte Rohre mit mindestens einer Durchbrechung in der Rohrwand. In diesem Fall muß zwischen Rohraußenwand und Silikonmembraninnenseite ein Netz oder Gewebe als Abstandshalter eingefügt werden, welches eine gleichmäßige Verteilung des aus dem Rohr durch die mindestens eine Durchbrechung zuströmenden Gases auf die Silikonmembran gewährleistet.

Bei Rohrmembranen, die einen auf den Hohlzylinder aufvulkanisierten Silikonfilm aufweisen, verschließt der Silikonkautschuk die Löcher derZyiinderwand. Erfindungswesentlich zur Erzielung guter Gasdurchlässigkeiten ist die Verwendung von Materialien der Dicke zwischen 0,05 mm und 1 mm für die auf die Zylinderwand aufzubringende Silikonkautschukschicht. Vorteilhaft ist dabei die Verwendung von Blechen mit runden, eckigen, oder länglichen Öffnungen der Größe 1 mm bis 3 mm oder von Metallgeweben mit Maschenweiten zwischen 0,05 mm und 2 mm. Die Summe der Durchbrechungsquerschnitte beträgt vorteilhaft 20% bis 70% der Zylinderwandfläche. Als Ausgangskomponenten können herkömmliche Silikonkautschukmischungen eingesetzt werden, die gegebenenfalls noch Zusätze enthalten, die die Haftung des Silikonfilms auf dem Material des Hohlzylinders verbessern. Die Viskositätseinstellung erfolgt gemäß den Angaben des jeweiligen Herstellers, falls erforderlich kann mit Lösungsmitteln zusätzlich verdünnt werden.

Erfindungsgemäße Silikonkautschukrohrmembranen erhält man weiterhin, wenn dünnwandiger Silikonschlauch der Dicke z.B. 0,2 mm auf den Hohlzylinder aufgebracht wird oder an der Hohlzylinderinnenwand angelegt wird. Vorteilhaft ist es in diesem Fall, den Silikonschlauch mit der Zylinderwand zu verkleben, wobei handelsübliche Silikonkleber verwendet werden können. Um mit Hilfe hoher Innendrücke hohe Gaseintragsraten zu erzielen, kann auf die gasdurchlässige Silikonmembran eine an sich bekannte Gewebeverstärkung als Druckträger aufgebracht werden. Vorteilhaft ist es weiterhin, eine zweite dünne Silikonmembran auf die Gewebeverstärkung aufzubringen. Diese zweite Silikonmembran hat die Aufgabe, Ablagerungen in der Gewebeverstärkung zu verhindern, sowie eine ggf. erforderliche Reinigung der Rohrmembran zu erleichtern. Zur weiteren Verbesserung der Druckbelastbarkeit ist es ferner möglich, die Silikonmembranen, Gewebeummantelungen, Abstandshalter und Hohlzylinder mittels geeigneter Silikonkleber miteinander zu verkleben. Die Gaszufuhr erfolgt an den Enden der Rohrmembran über entsprechend angepaßte Anschlußstutzen.

Die Vorteile der erfindungsgemäßen Rohrmembran aus gelochtem Blech, Metallgewebe oder gelochtem Kunststoff mit Silikonbeschichtung bzw. mit außen oder innen aufgeklebtem Silikonschlauch sind offenkundig wie das Beispiel einer silikonbeschichteten Rohrmembran aus Metallgewebe (Maschenweite 0,4 mm) der Länge 1000 mm und vom Außendurchmesser 30 mm zeigt. Auf das rohrförmige Stützelement wurde eine 0,2 mm dicke Silikonmembran aufgebracht und mit einer Gewebeummantelung versehen, auf die eine weitere 0,2 mm dicke Schutzummantelung aus Silikonkautschuk aufgebracht wurde. Aufgrund der spez. 0₂-Permeabilität einer derartigen Rohrmembran von 1000 mg 0₂ je Rohr und Stunde bei einem Sauerstoffpartialdruck von 5 bar im Rohrinnern werden zur Sauerstoffversorgung eines technischen Fermenters von 1 m³ Arbeitsvolumen lediglich ca. 50 Rohre mit einer Länge von 2 m benötigt, anstatt sonst erforderlicher 666 m verstärkten Silikonschlauchs.

Vorteilhaft ist die kreisförmige Ausrichtung dieser Rohre parallel zur Reaktorlängsachse. Die Anordnung kann in mehreren zueinander konzentrischen Kreisen erfolgen. Die Rohre werden oben und unten mit Kreisringen verbunden und können über diese mit Gas versorgt werden. Falls die Rohrmembranen parallel geschaltet werden sollen, ist es vorteilhaft, die Kreisringe aus Rohr herzustellen. Über ein Rohr wird das Gas wie z.B. Sauerstoff zugeführt und über das andere ein unerwünschtes Gas wie z.B. Kohlendioxid abgezogen. Desgleichen ist die Befestigung der Rohrmembranen auf einem zentralen Leitrohr möglich. Die Rohrmembranen können sowohl parallel als auch hintereinander geschaltet werden. Es ist auch der Gaseintrag in einen externen Flüssigkeitskreislauf eines Reaktors möglich. In einer weiteren Anwendung der erfindungsgemäßen Silikonrohrmembranen können zwei Rohrmembranen zu einer doppelseitig wirkenden Rohrflachmembran zusammengefügt werden. Hierfür werden zwei erfindungsgemäße Rohrmembranen von unterschiedlichem Durchmesser ineinander gestellt und stirnseitig gasdicht miteinander verbunden und mit den notwendigen Gaszu- bzw.-ableitungen versehen.

Weitere Merkmale der Erfindung werden in den abhängigen Ansprüchen beschrieben. In den Zeichnungen sind Beispiele für die Ausbildung und Anordnung von Rohrmembranen gemäß der Erfindung dargestellt, die nachstehend näher erläutert werden. Es zeigt
Fig. 1 die Ausbildung von parallel zueinander angeordneten Rohrmembranen in einer schematischen Seitenansicht,
Fig. 2 die Anordnung von Rohrmembranen in einer Reihenschaltung,
Fig. 3 die Ausbildung von doppelwandigen Rohrmembranen in einer schematischen Seitenansicht,
Fig. 4 und 5 die Ausbildung einer weiteren Rohrmembran in einer Seitenansicht im Schnitt und einer Draufsicht.

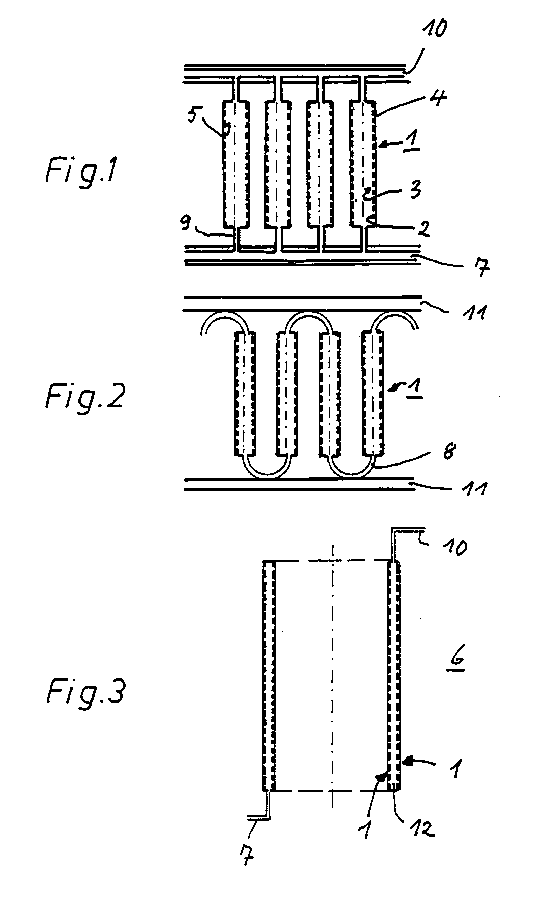

Bei der Anordnung nach Fig. 1 sind verschiedene Rohrmembranen 1 parallel zueinander angeordnet. Jede Rohrmembran 1 besteht aus einem Hohlzylinder 2 in dessen Hohlzylinderwand 5 Durchbrechungen 3 ausgebildet sind. Auf derAußenfläche der Hohlzylinderwand 5 befindet sich jeweils eine Silikonkautschukschicht 4. Jede Rohrmembran 1 ist mittels Anschlußleitungen 9 mit einer Gasversorgungsleitung 7 und einer Gasentsorgungsleitung 10 verbunden. Die Hohlzylinder 2 der Rohrmembran 1 können aus gelochtem Blech oder aus Metallgewebe bestehen.

Bei der in Fig. 2 schematisch dargestellten Anordnung befinden sich Rohrmembranen 1 in Reihenschaltung zwischen den Behälterwänden 11 z. B. eines Fermenters. Die Rohrmembranen 1 sind wie in Fig. 1 beschrieben ausgebildet. Die Verbindung der Rohrmembranen 1 untereinander erfolgt mittels Verbindungsleitungen 8.

In Fig. 3 ist schematisch ein Gasaustauscher 6 dargestellt, der aus zwei koaxial ineinander geschobenen Rohrmembranen 1 besteht. Zwischen den Rohrmembranen 1 ist eine kreisringförmige Kammer 12 ausgebildet, die mit einer Gasversorgungsleitung 7 und einer Gasentsorgungsleitung 10 verbunden ist. Mittels dieses Gasaustauschers ist es somit möglich, Gas beidseitig der Rohrmembranen 1 in die umgebende Flüssigkeit einzutragen. Es ist auch möglich, mehrere Gasaustauscher 6 mit unterschiedlichen Außendurchmessern ineinander zu schieben, wodurch ein konstruktiv besonderes kompakter, leistungsfähiger Gasaustauscher erzielt wird.

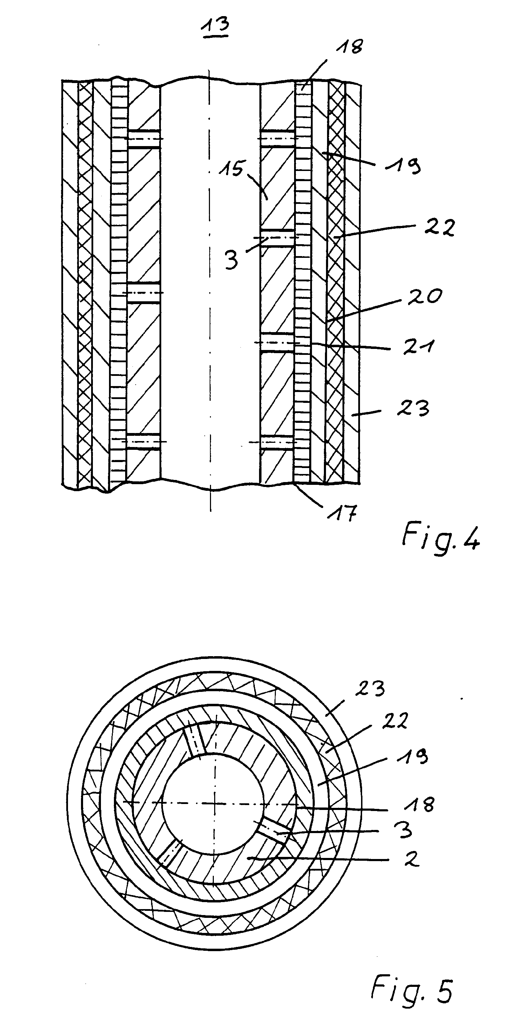

Die in Fig. 4 und 5 dargestellte Rohrmembran 13 weist als Tragelement einen mittigen Hohlzylinder 2 auf, dessen Durchmesser größer als 15 mm sein kann. In dem Zylindermantel 15 sind Durchbrechungen 3 ausgebildet. Auf der Außenfläche 17 des Hohlzylinders 2 ist ein gasdurchlässigerAbstandshalter 18 angeordnet. Auf dem Abstandshalter 18 ist eine Silikonmembran 19 aufgebracht, die sich mit ihrer Innenfläche 21 auf dem Abstandshalter 18 abstützt. Die Wanddicke der Silikonmembran 19 kann kleiner als 0,5 mm sein. Auf der Außenfläche 20 der Silikonmembran 19 ist eine Gewebeverstärkung 22 angeordnet. Diese ist von einer weiteren Silikonmembran 23 abgedeckt. Statt der Silikonmembran 23 kann auch eine Silikonkautschukschicht 4 aufgebracht werden. Die Silikonmembran 23 bzw. Silikonkautschukschicht 4 dient dazu, eine Verschmutzung an der Gewebeverstärkung 23 zu verhindern, die die Gasaustauschfähigkeit der Rohrmembran 13 beeinträchtigen würde. Statt eines Hohlzylinders 2 kann auch ein Drahtrohr als Tragelement verwendet werden. In diesem Fall wäre kein Abstandshalter 18 erforderlich. Die beschriebene Rohrmembran 13 kann in Längen von größer als 0,3 m hergestellt werden.

Die Rohrmembranen 1, 13 eignen sich für Gasaustauschprozesse in Behältern deren Behältervolumen größer als 0,05 m³ ist. Der Prozeß kann dabei so geführt werden, daß sich im Hohlzylinder 2 eine wässrige Flüssigkeit und zwischen der Behälterwand 11 und der Hohlzylinderwand 5 ein Gas oder Gasgemisch befindet. Es ist auch möglich, in den Hohlzylinder 2 ein Gas oder Gasgemisch einzubringen, wobei sich dann zwischen der Behälterwand 11 und der Hohlzylinderwand 5 eine wässrige Flüssigkeit befinden kann.

## Patentansprüche

1. Rohrmembran aus Silikonkautschuk mit einer mechanischen Wandverstärkung, dadurch gekennzeichnet, daß an einem Durchbrechungen (3) aufweisenden formsteifen Hohlzylinder (2) aus Metall oder Kunststoff eine gasdurchlässige Silikonkautschukschicht (4) mit einer Dicke von 0,05 mm bis 1 mm abgestützt ist.

2. Rohrmembran nach Anspruch 1, gekennzeichnet dadurch, daß sich die Silikonkautschukschicht (4) in den Durchbrechungen (3) der Hohlzylinderwand (5) befindet.

3. Rohrmembran nach den Anspruch 1, gekennzeichnet dadurch, daß sich die Silikonkautschukschicht (4) auf der Außenfläche der Hohlzylinderwand (5) befindet.

4. Rohrmembran nach Anspruch 1, gekennzeichnet dadurch, daß sich die Silikonkautschukschicht (4) auf der Innenfläche der Hohlzylinderwand (5) befindet.

5. Rohrmembran nach den Ansprüchen 1, 3 und 4, gekennzeichnet dadurch, daß die Silikonkautschukschicht (4) durch einen Silikonschlauch gebildet wird.

6. Rohrmembran nach den Ansprüchen 1, 3 und 4, gekennzeichnet dadurch, daß die Silikonkautschukschicht (4) aus einer gewebeverstärkten Silikonflachmembran besteht, die um den Hohlzylinder (2) gelegt und auf diesem befestigt ist.

7. Rohrmembran nach den Ansprüchen 1, 3 bis 6, gekennzeichnet dadurch, daß die Silikonkautschukschicht (4) mit der Hohlzylinderwand (5) des Hohlzylinders (2) verklebt oder vulkanisiert ist.

8. Rohrmembran nach den Ansprüchen 1 bis 7, gekennzeichnet dadurch, daß der freie Querschnitt der Hohlzylinderwand (5) zwischen 20% und 70% beträgt und der Durchmesser der Durchbrechungen (3) in der Hohlzylinderwand (5) größer als 0,1 mm ist.

9. Rohrmembran nach einem derAnsprüche 1, bis 8, dadurch gekennzeichnet, daß auf der Außenfläche (17) des Durchbrechungen (3) aufweisenden Hohlzylinders (2) ein gasdurchlässiger Abstandshalter (18) angeordnet ist, auf dem eine Silikonmembran (19) gelagert ist, die von einer anliegenden Gewebeverstärkung (22) umgeben ist.

10. Rohrmembran nach Anspruch 9, dadurch gekennzeichnet, daß auf der Gewebeverstärkung (22) eine Silikonmembran (23) oder Silikonkautschukschicht (4) angeordnet ist.

11. Verfahren zu Herstellung von Rohrmembranen nach Anspruch 1 aus Silikonkautschuk mit einer mechanischen Wandverstärkung, dadurch gekennzeichnet, daß ein Hohlzylinder aus Metall oder Kunststoff mit in derZyiinderwand ausgebildeten Durchbrechungen horizontal ausgerichtet mit Silikonkautschuk getränkt wird, dann durch Rotation des Hohlzylinders nicht mit diesem verbundener tropffähiger Silikonkautschuk durch Fliehkraft entfernt und dann der an dem Hohlzylinder haftende Silikonkautschukfilm die Durchbrechungen in der Zylinderwand des Hohlzylinders verschließend vulkanisiert wird.

12. Verfahren zur Herstellung von Rohrmembranen nach Anspruch 1 aus Silikonkautschuk mit einer mechanischen Wandverstärkung, dadurch gekennzeichnet, daß ein Silikonschlauch durch Beaufschlagung mit einem Unterdruck im Durchmesser erweitert wird, dann ein Hohlzylinder aus Metall oder Kunststoff mit in der Zylinderwand ausgebildeten Durchbrechungen in den Silikonschlauch eingeschoben wird, der danach durch Aufhebung des Unterdrucks auf den Hohlzylinder aufschrumpft und befestigt wird.

13. Verfahren zur Herstellung von Rohrmembranen nach Anspruch 1 aus Silikonkautschuk mit einer mechanischen Wandverstärkung, dadurch gekennzeichnet, daß ein Silikonschlauch durch Beaufschlagung des Schlauchinnenraums mit Unterdruck im Durchmesser verkleinert wird, dann in einen Hohlzylinder aus Metall oder Kunststoff mit in der Zylinderwand ausgebildeten Durchbrechungen eingezogen und dann durch Aufhebung des Unterdrucks an der Zylinderinnenwand zur Anlage gebracht und an dieser unter Druckbeaufschlagung verklebt wird.

14. Verfahren nach Anspruch 12 und 13, dadurch gekennzeichnet, daß ein Silikonschlauch mit einer Gewebeverstärkung verwendet wird.

## Claims

1. Tube membrane of silicone caoutchouc with a mechanical wall reinforcement, characterised in that a gas-permeable silicone caoutchouc layer (4) having a thickness of 0.05 mm to 1 mm is carried by a rigid hollow cylinder (2) of metal or plastics and has apertures (3) provided therein.

2. Tube membrane according to Claim 1, characterised in that the silicone caoutchouc layer (4) is located in the apertures (3) of the wall (5) of the hollow cylinder.

3. Tube membrane according to Claim 1, characterised in that the silicone caoutchouc layer (4) is located on the exterior surface of the wall (5) of the hollow cylinder.

4. Tube membrane according to Claim 1, characterised in that the silicone caoutchouc layer (4) is located on the internal surface of the wall (5) of the hollow cylinder.

5. Tube membrane according to Claims 1, 3 and 4, characterised in that the silicone caoutchouc layer (4) is formed by a silicone hose.

6. Tube membrane according to Claims 1, 3 and 4, characterised in that the silicone caoutchouc layer (4) consists of a fabric-reinforced silicone flat membrane, which surrounds the hollow cylinder (2) and is secured thereto.

7. Tube membrane according to Claims 1, 3 to 6, characterised in that the silicone caoutchouc layer (4) is glued to the wall (5) of the hollow cylinder (2) or vulcanised.

8. Tube membrane according to Claims 1 to 7, characterised in that the free cross-section of the wall (5) of the hollow cylinder ranges from 20% to 70% and that the diameter of the apertures (3) in the wall (5) of the hollow cylinder is greater than 0.1 mm.

9. Tube membrane according to any one of Claims 1, 3 to 8, characterised in that the exterior surface (17) of the hollow cylinder (2) having the apertures (3) has a gas-permeable spacer (18) arranged thereon, on which a silicone membrane (19) rests which is surrounded by an abutting fabric reinforcement (22).

10. Tube membrane according to Claim 1, characterised in that a silicone membrane (23) or silicone caoutchouc layer (4) is arranged on the fabric reinforcement (22).

11. Method of producing tube membranes according to Claim 1 from silicone caoutchouc with a mechanical wall reinforcement, characterised in that a hollow cylinder of metal or plastics, with apertures formed in the cylinder wall, is orientated in a horizontal direction and is impregnated with silicone caoutchouc, whereupon any silicone caoutchouc that is capable of dripping and is not connected with a hollow cylinder is removed by centrifugal force, forced by the rotation of said hollow cylinder, and the silicone caoutchouc film adhering to the hollow cylinder is vulcanised so as to close the aperture in the cylinderwall of the hollow cylinder.

12. Method of producing tube membranes according to Claim 1 of silicone caoutchouc with a mechanical wall reinforcement, characterised in that the diameter of a silicone hose is expanded by applying a vacuum, a hollow cylinder of metal or plastics having apertures in the cylinder walls is then pushed into the cylinder hose by removing the vacuum, shrunk onto the hollow cylinder and secured.

13. Method of producing tube membranes according to Claim 1 of silicone caoutchouc having a mechanical wall reinforcement, characterised in that the diameter of a silicone hose is reduced by applying a vacuum onto the interior of the hose, followed by pulling in a hollow cylinder of metal or plastics with apertures in the cylinder wall, and subsequently causing the hose to rest against the interior cylinderwall by removing the vacuum and adhering thereto by applying a pressure.

14. Method according to Claims 12 and 13, characterised in that a silicone hose having a fabric reinforcement is used.

## Revendications

1. Membrane de tuyau en caoutchouc au silicone muni d'un renforcement mécanique de paroi, caractérisée en ce qu'une couche de caoutchouc au silicone (4) perméable aux gaz, d'une épaisseur de 0,05 mm à 1 mm, s'appuie sur un cylindre creux (2) indéformable qui présente des découpures (3) et est fabriqué en métal ou en matière synthétique.

2. Membrane de tuyau selon la revendication 1, caractérisée en ce que la couche de caoutchouc au silicone (4) se trouve dans les découpures (3) de la paroi du cylindre creux (5).

3. Membrane de tuyau selon la revendication 1, caractérisée en ce que la couche de caoutchouc au silicone (4) se trouve sur la face extérieure de la paroi du cylindre creux (5).

4. Membrane de tuyau selon la revendication 1, caractérisée en ce que la couche de caoutchouc au silicone (4) se trouve sur la face interne de la paroi du cylindre creux (5).

5. Membrane de tuyau selon les revendications 1, 3 et 4, caractérisée en ce que la couche de caoutchouc au silicone (4) est formée par un tuyau flexible au silicone.

6. Membrane de tuyau selon les revendications 1, 3 et 4, caractérisée en ce que la couche de caoutchouc au silicone (4) se compose d'une membrane plate au silicone présentant une doublure de renfort arrière, membrane qui est placée autour du cylindre creux (2) et fixée sur celui-ci.

7. Membrane de tuyau selon les revendications 1, 3 à 6, caractérisée en ce que la couche de caoutchouc au silicone (4) est collée à la paroi (5) du cylindre creux (2) ou vulcanisée.

8. Membrane de tuyau selon les revendications 1 à 7, caractérisée en ce que la section transversale libre de la paroi du cylindre creux (5) se situe entre 20 et 70% et en ce que le diamètre des découpures (3) de la paroi du cylindre creux (5) est supérieur à 0,1 mm.

9. Membrane de tuyau selon l'une des revendications 1, 3 à 8, caractérisée en ce que, sur la face extérieure (17) du cylindre creux (2) présentant des découpures (3), est disposé un écarteur (18) perméable aux gaz sur lequel est logée une membrane au silicone (19) qui est entourée par une doublure de renfort arrière (22) collée.

10. Membrane de tuyau selon la revendication 9, caractérisée en ce qu'une membrane au silicone (23) ou couche de caoutchouc au silicone (4) est disposée sur la doublure de renfort arrière (22).

11. Procédé de fabrication de membranes de tuyau selon la revendication 1, en caoutchouc au silicone et munies d'un renforcement mécanique de paroi, caractérisé en ce qu'un cylindre creux en métal ou matière synthétique, aligné horizontalement avec des découpures formées dans la paroi du cylindre, est imprégné de caoutchouc au silicone, puis en ce que, par rotation du cylindre creux, du caoutchouc au silicone non relié à celui-ci et qui peut goutter est éliminé par force centrifuge et en ce qu'enfin le film de caoutchouc au silicone qui adhère au cylindre creux et qui obture les découpures de la paroi du cylindre creux est vulcanisé.

12. Procédé de fabrication de membranes de tuyau selon la revendication 1, en caoutchouc au silicone et munies d'un renforcement mécanique de paroi, caractérisé en ce que le diamètre d'un tuyau flexible au silicone est agrandi par admission d'un vide, puis en ce qu'un cylindre creux en métal ou en matière synthétique est introduit, à l'aide des découpures formées dans la paroi du cylindre, dans le tuyau flexible au silicone qui est ensuite ramené au diamètre du cylindre creux par suppression du vide et fixé sur ce cylindre creux.

13. Procédé de fabrication de membranes de tuyau selon la revendication 1, en caoutchouc au silicone et munies d'un renforcement mécanique de paroi, caractérisé en ce que le diamètre d'un tuyau flexible au silicone est diminué par admission de vide à l'intérieur du tuyau flexible, en ce que ce tuyau flexible est ensuite introduit dans un cylindre creux en métal ou en matière synthétique à l'aide de découpures formées dans la paroi du cylindre, puis amené en appui sur la paroi interne du cylindre par suppression du vide et collé sur celle-ci sous pressurisation.

14. Procédé selon les revendications 12 et 13, caractérisé en ce qu'un tuyau flexible au silicone muni d'une doublure de renfort arrière est utilisé.
